# EUROPEAN PATENT SPECIFICATION

(11) **EP 1 645 295 B1**
(45) Date of publication and mention of the grant of the patent: **18.04.2012**
(21) Application number: 05021860.1
(22) Date of filing: 05.10.2005
(51) Int. Cl.: A61L 9/12, H02G 3/08

(54) **Controlled-release diffuser for room deodorants and/or perfumes, designed to be wall-mounted**
Geregelter Spender von Raumbeduftungsmitteln und/oder Parfümen, der in eine Wand einbaubar ist
Diffuseur à distribution controlée pour déodorant et/ou parfum destiné à etre monté sur un mur

(30) Priority: 08.10.2004 IT mi20040454 U
(43) Date of publication of application: 12.04.2006
(73) Proprietor: OIKOS S.r.l., 20149 Milano (IT)
(72) Inventor: Bader, Stefano, 20121 Milano (IT); Verderio, Silvia, 20121 Milano (IT)
(74) Representative: Minoja, Fabrizio

(56) References cited:
- EP-A- 0 397 301
- EP-A- 0 645 148
- EP-A- 1 172 119
- EP-A- 1 356 728
- US-A- 4 452 500
- US-A- 4 666 638
- US-A- 4 707 338
- US-A- 5 419 879
- US-A1- 2003 124 022

## Description

This invention relates to a controlled-release diffuser for room deodorants and/or perfumes, which comprises a container for the perfuming product fitted with means designed to direct air onto the product and introduce it into the room, which said container is equipped with connectors designed to allow it to be inserted into a box built into the wall, the assembly being of such dimensions and configuration as to allow it to be inserted into the ordinary type of back box used in electrical installations.

The container according to the invention can therefore be mounted in a back box similar to those used for electrical installations, and then completed with the same faceplate as used for switches and other electrical devices, so that it matches the other appliances present in the home.

In particular, the diffuser according to the invention comprises a cartridge into which pastilles containing the fragrance are inserted, such as pastilles obtained by compressing silicon dioxide powder or another natural and/or synthetic porous base mixed with a suitable quantity of fragrance, said cartridge being inserted in a back box closed at the sides, the base of which is fitted with a fan that directs air onto the pastilles of product and causes it to exit from a front grid.

The diffuser is designed in such a way that the power supply to said fan can be connected to the timer or the units that control the operation of the other electrical and electronic devices in the home, in order to regulate the emission of perfume as required.

Different types of diffuser are known which release a perfume or deodorant into a location which could be a room of a home, a vehicle interior or the like.

Many of these devices diffuse the perfume into the room by evaporation of the product.

As the fragrances are generally constituted by oils which have a fairly high boiling point and/or vapour pressure, in order to allow evaporation at a temperature just above room temperature, said oils are mixed with solvents, producing a liquid composition which is used in association with diffusers constituted by a container fitted with a resistor that heats the composition gently to promote dispersal of the perfume into the room.

These are basically devices wherein a complex formulation is caused to evaporate by means of kinetics due to a change of state resulting from a temperature increase, with the result that evaporation and the extent of vapour diffusion cannot be easily controlled or modulated.

These diffusers need to be very cheap; it is not economical to equip them with temperature control sensors or devices, so a low-powered resistor that always develops the same amount of heat is fitted.

If the container contains a small amount of product, the product will reach a higher temperature, diffusing a larger quantity of perfume than when the container is full of liquid.

The diffusion of the perfume also depends on other factors, such as environmental humidity, etc., which not are easily controllable.

Another type of diffuser diffuses the perfume into the room by spraying a liquid that contains the fragrance.

Part of the liquid remains suspended in the air and part falls to the ground, with the result that the effect and extent of perfuming varies, depending on whether people are moving about in the room, for example.

Systems are also known which involve sprinkling into the room microcapsules containing perfume that break when they are trodden on, releasing the fragrance.

Once again, the effect is obviously not controllable.

Basically, systems which involve diffusion of a perfume mixed with a solvent cannot easily be modulated, because even if the diffusion of the solvent in the air could be controlled, it is difficult to control how the solvent releases the fragrance.

EP-A-0.645.148 describes an air freshening device, to be mounted on a wall 50, which comprises a housing 10 containing a perfume cassette (30), a fan 20, a perfume cassette 30 and an electric circuit 40. The housing 10 has a perforated lid 11 and base 12. Means are provided to fix the back panel to the wall 50.
The perfume cassette 30 is a solid block of perfume material having vertical channels through it.
The fan 20 directs air onto the perfume material and then into the room.
EP-A-0.397.301 relates to a battery operated air freshener device which includes a housing having means for mounting the air freshener device to surface such as a wall, a motor connected to a fan and to a battery, a replaceable cartridge with vents defining an air flow path for carrying a deodorizing substance.
US 4.666.638 shows a battery operated fragrance device, which establishes an air stream over a fragrant material when an artificial light source such as a room lamp is energized. The unactuated device provides a limited flow of convection air current which maintains a relatively low level of fragrance dispersion in a room. Upon actuation forced air flowing through the device causes a rapid increase in the level of fragrance dispersion in the room.
US 5.419.879 relates to a perfumed stable gel comprised of a combination of a chemical components. The perfumed gel may be retained in a disposable deodorant container which is adapted for use with various deodorant dispensers
US 4.452.500 relates to a deodorant cabinet wherein a cover is hinged to a back plate. The cabinet is opened by insertion of a key and further includes a fan that draws air past deodorant material on shelf and expels it through louvers.
US 4.707.338 describes an aroma generator which discharges an air current conveying an aromatic vapor. A motor-driven fan forces air through an air-permeable cartridge containing an aroma supply.
EP-A-1.356.728 too, shows a fan type chemical diffusing apparatus.

The present innovation, which falls into this sector, relates to a controlled-release diffuser for deodorants and/or perfumes designed for rooms which uses pastilles constituted by a compressed powder mixed with a perfume or fragrance and has a configuration allowing it to be inserted into the ordinary back boxes used for electrical devices, the diffuser having the feature listed in appended independent claim 1.

The result is a practical diffuser which is easy to instal, the base of which is fitted with a fan that directs air onto the perfuming product and expels it into the room.

The release of the fragrance can thus be easily controlled simply by regulating the number of pastilles in the cartridge, the fan operating cycles, and possibly the fan speed.

This innovation will now be described in detail, by way of example but not of limitation, by reference to the annexed figures wherein;
- figure 1 is a front view of a diffuser not according to the invention, assembled and wall-mounted, without the faceplate;

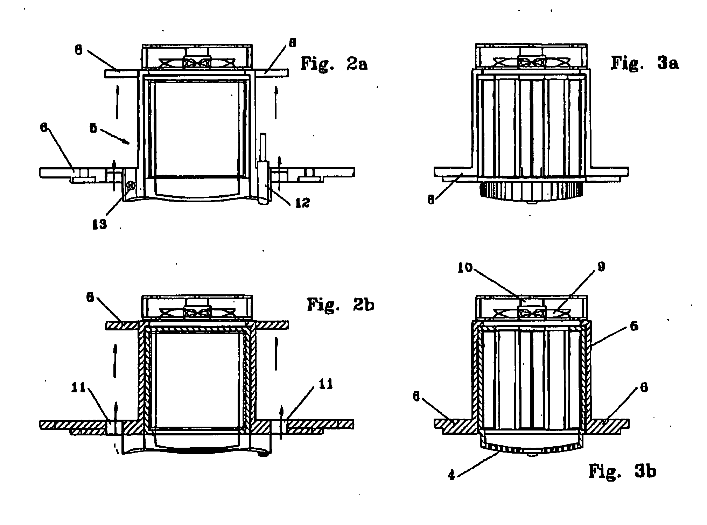

- figures 2a and 2b are the view from above and cross-section respectively of a diffuser, also wall-mounted;
- figures 3a and 3b are the side view and cross section of a wall-mounted diffuser;
- figures 4, 5, 6 and 7 are the front view, front cross-section, view from above and side view respectively of the diffuser cartridge;
- figure 8 is a lateral cross-section of the diffuser cartridge.

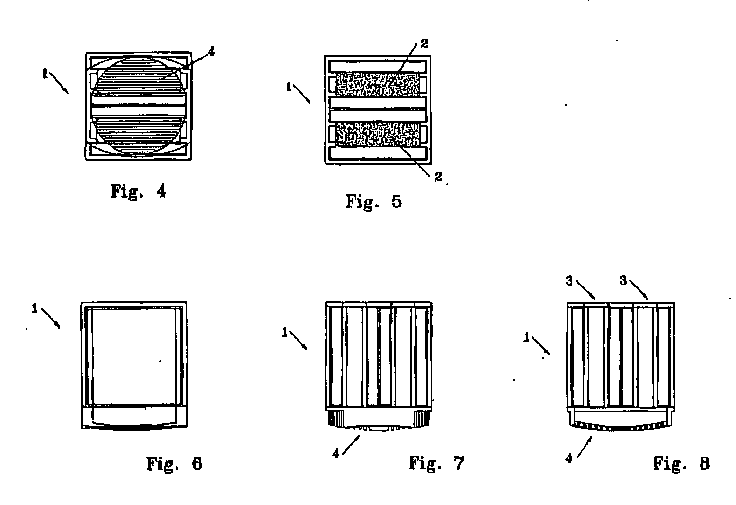

The diffuser comprises a cartridge, shown as no. 1 in figures 4 to 7, into which are inserted one or more pastilles 2 (preferably 2 or 4 pastilles) of a perfuming product constituted, for example, by a silicon dioxide powder mixed with a fragrance and suitably compacted to obtain the pastilles.

The back wall of cartridge 1 contains a series of openings 3 for the entry of air designed to diffuse the perfume into the room, while on the opposite side there is a perforated grid 4, the design of which can vary, depending on the desired appearance of the device.

Cartridge 1 is designed to be inserted into a frame 5, fitted on the front with a flange 6 or the like designed to allow it to be connected to a box built into the wall, preferably constituted by a standard back box of the type used for electrical installations.

On the bottom of frame 5 there is a support base 8 for the electronic circuit designed to control the device, and a fan 9 or the like, driven by an electric micromotor 10.

Fan 9 sucks in air from the base and conveys it into cartridge 1, inserted into frame 5, so that the air then exits from grid 4 and is dispersed into the room.

Front flange 6 of frame 5 contains holes 15 so that it can be secured with screws or the like to the connectors commonly present on back boxes, and is shaped to allow snap-on fitting of a faceplate not illustrated in the figures, which is applied to flange 6 after the diffuser has been fitted.

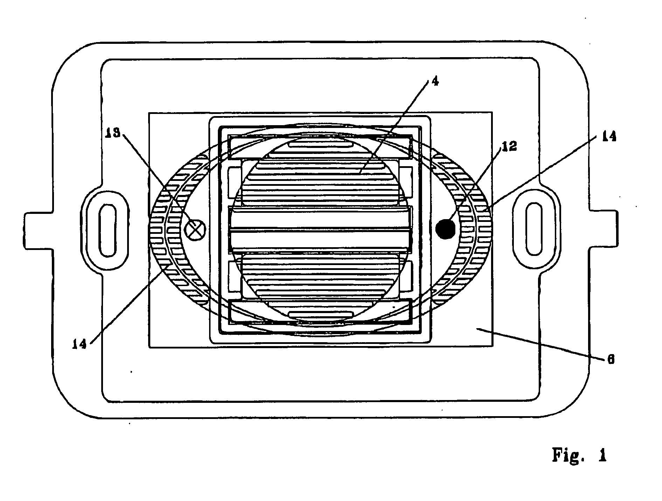

At the sides, flange 6 presents a pair of grids 14 (Fig. 1) through which fan 9 sucks in air to be conveyed towards the perfuming product.

A pushbutton 12 used to switch on and regulate the device is incorporated on one side of flange 6, and a device such as a LED 13 or the like which indicates when the device is on may be fitted on the opposite side.

Pushbutton 12 can directly control the power supply to fan 9, or said fan can be connected, via an electronic contact and wires not illustrated in the figure, to an external control unit, which may be a specific unit or more generally the unit that controls the electrical and electronic devices in the home (the home automation control unit which, for example, manages the heating installation, safety devices, alarms, lighting, etc.).

To fit the diffuser it is sufficient to connect the power cables, fit frame 5 into the back box, fix flanges 6 to its connectors, and complete the assembly if required by fitting a faceplate.

To diffuse the perfume into the room, it is sufficient to insert cartridge 1 containing pastilles 2 and operate pushbutton 12 to switch on the motor of fan 9.

This will cause air to be sucked in from the outside through grids 14 and inlets 11 in flange 6, conveyed from the base of the container onto the pastilles of perfuming product, and returned to the room through front grid 4.

If it is decided to change the fragrance, or the perfuming pastilles are used up, it is sufficient to replace the cartridge with another one of the desired perfume.

Pushbutton 12 (or possibly a remote control) could be used merely to switch the diffuser on and off, or also to control its various functions.

By way of example, a short pressure on the pushbutton could operate the fan, while suitably coded pressure durations could be used to programme the device with different control and operation functions (cycles, speed, duration, etc.). The result of the programming will be displayed by LED 13 using suitable visual codes.

The operation of the device could be controlled in an even more powerful, versatile way by a remote control unit.

Grid 4 and side grids 14 could be made with different designs, to give the product a particularly attractive appearance.

As air is sucked in at the sides and sent back into the room from the centre of the diffuser, an air circulation is created that promotes the diffusion of perfume in the room.

The device could be activated and deactivated by successively pressing pushbutton 12, the state of operation being indicated by LED 13; alternatively, the device could be connected, for example, to a timer or control unit which allows its operation to be programmed for pre-set times and periods.

## Claims

1. Controlled-release diffuser for deodorants and/or perfumes designed for rooms, the diffuser including
a cartridge (1) designed to receive pastilles (2) of a deodorizing and/or perfuming product, said cartridge (1) presenting air inlets (3) and an air outlet grid (4);
the diffuser further including a frame (5) with closed side walls designed to house said cartridge (1) and with a support base (8) at the bottom thereof;
said base (8) being fitted with a fan (9) designed to direct air onto the product in the cartridge and then into the room;
**characterized in that** said frame (5) further comprises a flange (6) being integral with said frame (5) and equipped with systems designed to allow it to be fixed to a wall mounted standard electrical installation back box.

2. Diffuser as claimed in claim 1, **characterised in that** it includes, on the wall in contact with the room, a central opening for the passage of perfumed air to be emitted into the room, and one or more side openings **(14)** for the entrance of air to be directed towards the perfuming product and then into the room.

3. Diffuser as claimed in claim **1, characterised in that** said flange **(6)** presents side openings **(14)** for suction of air to be directed towards the perfuming product.

4. Diffuser as claimed in claim **1, characterised in that** the base **(8)** of said frame is fitted with an electronic card on which are mounted electronic devices designed to allow the local or remote management and control of the device.

5. Diffuser as claimed in the preceding claims, **characterised in that** it includes a fan activation pushbutton **(12).**

6. Diffuser as claimed in claim **5, characterised in that** it includes luminous means **(13)** designed to display the operation of the fan.

7. Diffuser as claimed in the preceding claims, **characterised in that** it includes means for connection to a control unit to allow programming of the operation of the device.

## Patentansprüche

1. Verteiler mit gesteuerter Freigabe für Deodorants und/oder Parfüms, die für Räume konzipiert sind, wobei der Verteiler Folgendes aufweist
eine Patrone (1), die konzipiert ist, um Tabletten (2) eines desodorierenden und/oder parfümierenden Produkts aufzunehmen, wobei die Patrone (1) Lufteinlässe (3) und ein Luftauslassgitter (4) aufweist;
wobei der Verteiler ferner einen Rahmen (5) mit geschlossenen Seitenwänden aufweist, um die Patrone (1) aufnehmen zu können, und mit einer Tragbasis (8) an seinem Boden konzipiert ist;
wobei die Basis (8) mit einem Ventilator (9) ausgestattet ist, der dazu konzipiert ist, Luft zu dem Produkt in der Patrone und dann in den Raum zu lenken;
**dadurch gekennzeichnet, dass** der Rahmen (5) ferner einen Flansch (6) aufweist, der mit dem Rahmen (5) integral und mit Systemen ausgestattet ist, die konzipiert sind, um zu erlauben, ihn an einer an einer wandmontierten elektrischen Standardeinbaudose zu befestigen.

2. Verteiler nach Anspruch 1, **dadurch gekennzeichnet, dass** er auf der Wand in Berührung mit dem Raum eine zentrale Öffnung für das Durchgehen parfümierter Luft, die in den Raum abzugeben ist, aufweist, und eine oder mehrere Seitenöffnungen (14) für das Eintreten von Luft, die zu dem parfümierenden Produkt und dann in den Raum zu lenken ist.

3. Verteiler nach Anspruch 1, **dadurch gekennzeichnet, dass** der Flansch (6) Seitenöffnungen (14) zum Ansaugen von Luft, die zu dem parfümierenden Produkt zu lenken ist, aufweist.

4. Verteiler nach Anspruch 1, **dadurch gekennzeichnet, dass** die Basis (8) des Rahmens mit einer Elektronikkarte ausgestattet ist, auf die elektronische Vorrichtungen montiert sind, die konzipiert sind, um das lokale oder dezentrale Verwalten und Steuern der Vorrichtung zu erlauben.

5. Verteiler nach dem vorhergehenden Anspruch, **dadurch gekennzeichnet, dass** er eine Drucktaste (12) zum Betätigen des Ventilators aufweist.

6. Verteiler nach Anspruch 5, **dadurch gekennzeichnet, dass** er Leuchtmittel (13) aufweist, die dazu bestimmt sind, den Betrieb des Ventilators anzuzeigen.

7. Verteiler nach den vorhergehenden Ansprüchen, **dadurch gekennzeichnet, dass** er Mittel zum Verbinden mit einer Steuereinheit aufweist, um das Programmieren des Betriebs der Vorrichtung zu erlauben.

## Revendications

1. Diffuseur à distribution contrôlée pour déodorants et/ou pour parfums, conçu pour des locaux, le diffuseur incluant
une cartouche (1) conçue pour recevoir des pastilles (2) d'un produit désodorisant et/ou parfumant, ladite cartouche (1) présentant des entrées d'air (3) et une grille de sortie d'air (4) ;
le diffuseur incluant en outre un cadre (5) avec des parois latérales fermées conçues pour loger ladite cartouche (1) et avec une base de support (8) au niveau de sa partie inférieure ;
ladite base (8) étant équipée d'un ventilateur (9) conçu pour diriger de l'air sur le produit dans la cartouche et ensuite dans le local ;
**caractérisé en ce que** ledit cadre (5) comprend en outre une bride (6) réalisée de façon monobloc avec ledit cadre (5) et qui est équipée de systèmes conçus pour lui permettre d'être fixée sur un boîtier arrière d'installation électrique standard monté sur une paroi.

2. Diffuseur selon la revendication 1, **caractérisé en ce qu'**il inclut, sur la paroi en contact avec le local, une ouverture centrale pour le passage d'air parfumé à émettre vers le local, et une ou plusieurs ouvertures latérales (14) pour l'entrée de l'air qu'il s'agit de diriger vers le produit parfumant et ensuite vers le local.

3. Diffuseur selon la revendication 1, **caractérisé en ce que** ladite bride (6) présente des ouvertures latérales (14) pour la succion de l'air qu'il s'agit de diriger vers le produit parfumant.

4. Diffuseur selon la revendication 1, **caractérisé en ce que** la base (8) dudit cadre est équipée d'une carte électronique sur laquelle sont montés des dispositifs électroniques conçus pour permettre la gestion locale ou à distance et la commande du dispositif.

5. Diffuseur selon les revendications précédentes, **caractérisé en ce qu'**il inclut un bouton-poussoir (12) pour l'activation du ventilateur.

6. Diffuseur selon la revendication 5, **caractérisé en ce qu'**il inclut des moyens lumineux (13) conçus pour mettre en évidence le fonctionnement du ventilateur.

7. Diffuseur selon les revendications précédentes, **caractérisé en ce qu'**il inclut des moyens de connexion à une unité de commande afin de permettre la programmation du fonctionnement du dispositif.
